# EUROPEAN PATENT SPECIFICATION

(11) **EP 1 216 673 B1**
(45) Date of publication and mention of the grant of the patent: **26.10.2005**
(21) Application number: 00127793.8
(22) Date of filing: 19.12.2000
(51) Int. Cl.: A61F 13/42

(54) **Absorbant article with wetness indicator**
Absorbierender Artikel mit Feuchtigkeitsanzeige
Article absorbant possédant un indicateur d'humidité

(43) Date of publication of application: 26.06.2002
(73) Proprietor: SCA Hygiene Products AB, 405 03 Göteborg (SE)
(72) Inventor: Kling, Robert, 51163 Skene (SE)
(74) Representative: Harrison, Michael Charles

(56) References cited:
- EP-A- 0 776 645
- DE-A- 19 833 485
- US-A- 3 675 654
- US-A- 4 327 731
- US-A- 4 931 051
- US-A- 5 089 548
- US-A- 6 060 256
- US-A- 6 160 198

## Description

### Field of the invention:

The present invention relates to a gate system for an absorbent product, wherein said absorbent product is of the type intended for the absorption of bodily exudates. In particular, the invention relates to a gate system comprising a gate means coupled to at least a part of an indicator means, for use in diapers or other absorbent garments of a disposable or re-usable type, as defined in the preamble of claim 1.

### Background to the invention:

Document US-A-4 231 370 discloses the use of a pH indicator in absorbent products, whereby the presence of wetness by urine can be detected. In particular, the use of a pH-sensitive coating indicator layer is proposed, by means of which urine comes into direct contact with, and can cause a change in colour of the indicator. When urine of pH 6.2 was used with a specific indicator, a colour change was observed within about 5 minutes. Lower pH values give a slower change whilst higher pH values give a faster change. Thus, wetness at all normal pH values is detected, merely with differing speed. Urine is disclosed as having a pH varying between 4.6 to 8.0, whilst having an average pH of about 6.0.

While wetness can be detected in US-A-4 231 370, the indication system is unable to provide a selected detection threshold which is dependent on the presence of wetness alone or a threshold which is pH-specific. Instead, the system simply displays wetness according to a non-predetermined time relationship at all urine pH values. Clearly this is disadvantageous, since the presence of a small amount of urine of high pH value will also cause an indication that the absorbent product should be changed, even though a change is not necessarily required due to there being only a small quantity of urine present. Additionally, this small amount of urine will be stored in the absorbent core of the product away from the bodyside layer (i.e. top sheet) and thus in a position remote from, and non-harmful to, the skin. Such an absorbent product indication system is therefore subject to over-frequent indications of the need to change the absorbent product. Typically such "false" indications result in the person responsible for changing the absorbent product simply ignoring the indicator and thereby negating its purpose.

It is also known that incontinence events can be detected in absorbent pads by wetness detection systems as in US-A-5 537 095. This document discloses wetness being detected at different parts of an absorbent pad placed on a bed. The system operates by using a plurality of electrical circuits embedded in the pad which are short-circuited due to the presence of fluid. The short-circuits are then detected by a control unit including a series of multiplexers. The control unit is positioned externally to the pad and the circuits inside the pad remain unaltered. When a certain number of short-circuits is detected, a signal is produced in the control means indicating that a specific minimum volume of liquid is deemed present. The control system is thus activated upon reaching a detected threshold level set in the external control circuitry. The system is relatively expensive due to the use of complex electronic components. Additionally, the system relies on the threshold and control circuitry located outside the absorbent pad, which must be connected to the pad by a mechanical plug/connector system outside the pad system.

Document US-A-6 160 198 discloses diaper including a gate system in accordance with the preamble of claim 1, in which a range of values, including moisture, PH, conductivity and a whole range of other variables, can be used for gate activation. The document also mentions polymer films that can open to allow an actuator to release stored energy to perform a responsive function.

Document DE-A-198 33 485 discloses a detection means within an absorbent product, whereby using a transfer means, such as a fluid, a detector in a wettable area of the product causes an indication at an area remote from the detection point.

The soiling of diapers and other absorbent products is perhaps most commonly due to the presence of urine and/or faeces in such articles. In particular when the presence of faeces is detected, the product should be removed and replaced by a clean one. The soiled product may be discarded if it is the disposable type, or be cleaned and used again if it is of the reusable type.

In infants, diapers are changed typically between four and eight times during a twenty-four hour period. With adults, the frequency of changing incontinence garments such as diapers is often left to the adult's discretion based on a level of comfort or convenience, although when the wearer is bed-ridden, changing frequency often depends on the attendant personnel. Diapers in particular are often not changed at an appropriate time when viewed in terms of the damage that may occur to a wearer's skin not only by the presence of urine alone, but in particular when urine and faeces are together in the diaper. This condition may often remain undetected for several hours, especially when the wearer exhibits no immediate signs of discomfort.

With babies for example, whose skin is particularly sensitive, diapers are often changed when the baby cries or otherwise indicates discomfort, or upon specifically checking the diaper for the presence of excess wetness and/or faeces. The checking process itself may often be misleading however, since an attendant often relies on an inspection by stretching open one leg elastic only, such that some faecal and urine discharges often go undetected.

Whilst urine is typically quickly absorbed and often held separate from the wearer's skin in an absorbent core, faeces is generally maintained against the skin since it does not pass through into the core, unless special provision is made for this in the top sheet design. When faeces is present first and then followed by urine, the urine itself may also be held in contact with the skin by the faeces. The presence of urine and faeces together is however recognised by the present inventors to be particularly harmful to the skin and a problem which should also be alleviated wherever possible.

This contact with the skin, and the presence of urine and faeces together, is harmful to the skin since this combined presence results in a chain of events causing breakdown of the skin. Faecal bacteria namely have urease which will react with urea from urine to thereby create ammonia. The presence of ammonia in the diaper will, in turn, raise the pH value and cause faecal protease and lipase to become more active, this activity increasing further with increasing pH. It is this activity which contributes to the wearer's skin becoming progressively and cumulatively damaged and leaves it at far greater risk to infections.

As will be apparent from the above, this is one of the reasons why nappy rash (diaper rash) and skin infections occur in body areas covered by absorbent products. Thus, whilst visual checks are made on the wearers of such products at specific or random intervals, the products in some cases (e.g. when simply wet) may in fact be less harmful even when left for long periods as compared to when urine and faeces are present together even for only relatively short periods.

Although the systems described in the aforementioned prior art documents are sensitive to wetness by urine, a means is still required which can more specifically detect the presence of specific substances and/or specific properties of such substances in absorbent products, whilst at the same time not requiring the use of external detection equipment, and which also at the same time provides a system which helps to prevent false detections. The present invention is aimed at solving these problems.

By providing such a means, this can be used to further reduce the possibility of skin damage, based on the recognition of harmful conditions within an absorbent product, to thereby allow said product to be changed when these conditions indeed arise and not at too early or late a stage.

It is a secondary object of the invention that the means should be cheap to both manufacture and to include in an absorbent product, such that the required means can be made disposable together with a disposable absorbent product.

### Summary of the invention

The main objective is achieved by an absorbent product having the features defined in claim 1. Preferred features of the invention are defined in the dependent claims.

Further features of the invention will be apparent to the reader of this specification.

In accordance with the invention, the gate means functions as a result of its contact with body exudate, for example by urine or faeces being in direct contact with the gate. A further possibility provided by the invention is however that a product of the exudate, such as faecal gas (e.g. containing mercaptans or the like) or ammonia etc., can contact and thus operate on the gate to change its state.

An indicator means of the invention could comprise a detector portion such as a sensor within a diaper, connected to an indicator portion such as a visible or audible sign outside a diaper. The indicator portion can be spaced from the detector portion.

The gate means of the invention could thereby be arranged to prevent activation of either the detector portion and/or of the indicator portion. For example, since the gate means is a dissolvable substance, it could be coated on the detector portion so as to prevent contact of exudate with the detector until it has been in contact with the gate means for a predetermined time. Similarly, the indicator means could comprise a combined detector and indicator (e.g. a combined pH-detector/visual indicator), whereby the gate could be used to cover or encapsulate the combined indicator. Thus it will be apparent that the gate means should be coupled to at least a part of the indicator means, this part being typically the detector portion or the indicator portion of same. In other cases it could be coupled to a connection means joining the detector portion and the indicator portion (if such a connection means is present).

The gate means itself is normally in its passive or rest state when no contact with body exudate has been made. This normal or rest state is herein referred to as the first state. The gate remains in this state until reaching a predetermined threshold. Once this threshold is exceeded, the gate changes to a second state or, in other words "opens". The threshold chosen depends on the parameter being evaluated for deciding when an indication to an observer should be provided. In the case of the invention, the predetermined threshold includes at least a pH threshold.

Similarly, a combination of parameters, including a pH threshold, might be used, such as pH and time combined, whereby a predetermined pH level would have to be reached and maintained for a certain predetermined time before the gate would change from its first state to its second state.

In this way, an indication can be provided based on a predetermined set of conditions. This also allows an absorbent product designer the possibility of varying the predetermined gate parameters so as to suit different groups of user and size of product, such that infant diapers may be provided with a gate threshold set at a different level (e.g. pH level, or pH level and time period) to those of an adult.

As mentioned in the introduction, the pH value of urine present in a diaper can be a particularly important factor for determining the presence of undesirable conditions. Since the pH threshold is used to determine when the gate is to switch between first and second states, a selected pH threshold level could be predetermined to be at a level of 6.5 or 7.0 for example, before the exudate (or product thereof) is able to activate the indicator means.

To avoid too rapid an indication for the case where only a small quantity of urine of high pH value is present (which may pass through the bodyside layer and into the absorbent core in a matter of seconds) a predetermined time factor can be built into the gate. When taking the gate of the invention made of a pH-sensitive terpolymer, i.e. a substance which is arranged to break down only after a specific pH level is reached, the time aspect can be accounted for by adjusting the thickness of the terpolymer. Thus when the predetermined pH threshold is exceeded, the terpolymer gate starts to dissolve but further requires contact with the urine for a specific (selected) time (e.g. several minutes) before dissolving to allow activation of an indicator portion. In this way, if the urine is of such a quantity that it is absorbed into the core and thus kept away from the terpolymer gate, the gate will remain sufficiently intact to prevent activation of the indicator means, whilst when sufficient urine is present around the area of the gate (i.e. due to a large amount of urine present), the gate means will be continuously broken down by the urine so as to allow the indicator means to be activated when the selected time has elapsed.

Additionally, it is possible to provide a series of gates as the gate means, whereby a first gate could have a first threshold (e.g. a time threshold of one minute) and a second gate could have a different threshold (e.g. a time threshold of ten minutes). Each of these gates could be provided in parallel with separate indicator portions, whereby, for example, a first indication might be made after urine of pH 7.0 or above is present for one minute followed by a separate indication when urine of pH 7.0 or above is present for ten minutes. These separate indications might be made by different colour indicators becoming visible. Thus an attendant could be warned by the first indication that a diaper change is merely "advisable" and by the second indication that a diaper change is "important".

In other embodiments it is imaginable that more than two indicators can be used and different types of gates may be used for detecting different exudates or other parameters.

A gated indicator means may also be used together with a non-gated indicator means. For example, a simple non-gated wetness indicator as described in the prior art in the introduction could be used to indicate the presence of wetness, whereas the gate means of the invention may be set to selectively allow an additional indicator means to be activated when a pH value is above the predetermined threshold pH value. In this way the gate means would allow activation of the additional indicator means when potentially harmful conditions exist for the skin.

The gates could also be arranged in series rather than parallel, whereby a first gate could provide the first contact medium to the exudate and would need to change to its second state in order to allow access of the exudate (or a product thereof) to the next gate.

Where a plurality of gates is used, the gates could be of different types. For example a first gate might be the pH-sensitive gate of the invention and another might be gas sensitive, or one time sensitive and another pH-sensitive. Further gates may also be used. A plurality of different indicators can also be used with a plurality of gates such that the indicators might be one visible and one audible, or different colour indicators, or other types of indicator(s).

It will be appreciated that the present invention can be used in absorbent products in which the exudates urine and faeces are typically present. Such absorbent products include especially diapers (for adult, infant and baby use), absorbent pants having an absorbent core (e.g. so-called training pants) as well as diaper chassis members and other incontinence products which have replaceable absorbent inserts. The invention may also find application in various other types of hygiene articles, such as sanitary napkins and the like.

### Brief description of the drawings

The invention will now be explained in more detail with reference to certain non-limiting embodiments thereof and with the aid of the accompanying drawings, in which:
- Fig. 1: shows a plan view of a diaper having a gate means, whereby the liner has been removed,
- Fig. 2: shows a cross-sectional view on to line II-II of Fig. 1
- Fig. 3: shows a cross-section similar to that in Fig. 2 with a modified form of indicator and gate means,
- Fig. 4: shows a schematic diagram illustrating an indicator means provided with a modified form of gate means,
- Fig. 5: shows an indicator means with a dye encapsulated by a gate means,
- Fig. 6: shows a cross-sectional view through a diaper in the flat state depicting an indicator means with an externally located indicator portion,
- Fig. 7: shows an embodiment of parallel gate means and associated indicators,
- Fig. 8: shows an embodiment of gate means in series with a single indicator, and
- Fig. 9: shows an embodiment using a gas threshold gate means.

### Detailed description of preferred embodiments:

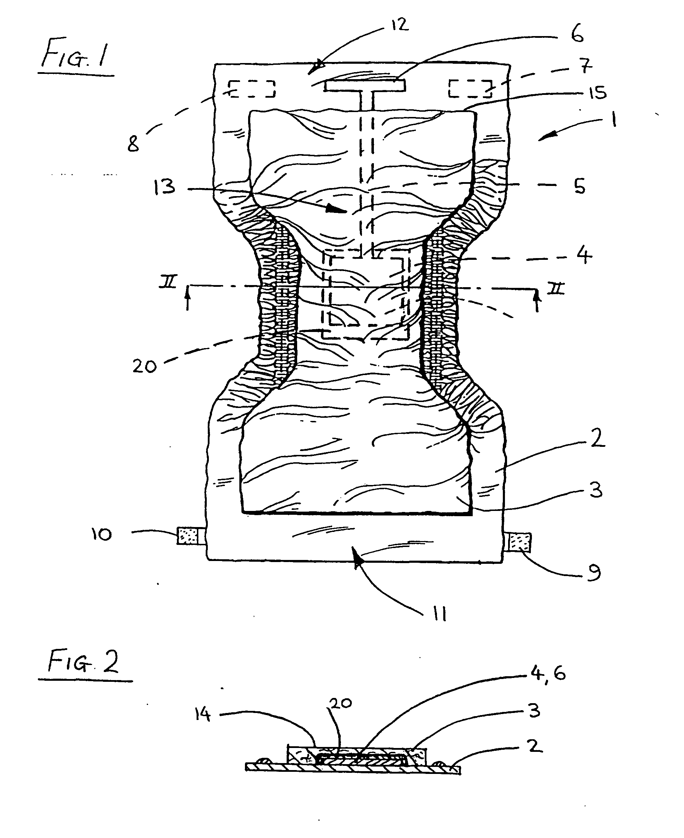

In Fig. 1, the absorbent product 1 in the form of a diaper has been illustrated in a flattened form and is depicted from the inner side thereof. For reasons of clarity, the top or cover sheet, sometimes referred to as a bodyside liner, has been removed.

Reference numeral 2 denotes a back sheet of liquid-impermeable material, typically consisting of a semi-transparent PE film. Such back sheet materials are common in the field of current day diapers and allow the underlying elements to be visibly discerned to a certain degree.

An absorbent core 3 of, for example, cellulosic material such as wood pulp fibres or the like, preferably mixed with a superabsorbent polymer, typically in an hour glass form, is positioned between the top sheet (not shown) and the back sheet 2. The absorbent core may be enveloped by a thin pervious layer such as a non-woven sheet (not shown) in some cases. The top sheet and back sheet 2 are then sealed around their contacting peripheries, preferably by means of welding or adhesive, to thereby form an envelope enclosing said core 3 and, if present, its enveloping pervious layer. The core 3 thus normally has dimensions which are smaller in both the length and width directions of the article, as shown.

Attachment means, such as hook and loop type mechanical fastening means, are provided at respective waist ends 11 and 12 of the article, whereby end 12 is intended to be the front waist portion of the product 1 and end 11 the rear waist portion, when applied to the wearer. In such an arrangement the tabs 7 and 8 could for example form loop tabs which are placed on the outer side of the back sheet 2, whilst tabs 9 and 10 could be hook tabs designed to engage with respective tabs 7 and 8 after the product has been passed through the legs of a wearer and up to the waist for attachment around the waist as is normal in the art.

An indicator means is attached to e.g. the back sheet 2 of the diaper and is denoted generally by numeral 13. As further shown in Fig. 2, the indicator means 13 has one end thereof placed in contact with the absorbent core 3 in a generally centrally located portion, typically the crotch portion. The opposite end of said indicator means is, in this embodiment, shown lying at the front waist region 12 of said product 1, outside the end edge 15 of the core. The entire indicator means 13 is thus positioned on the inner, core-facing side of the back sheet 2 in this particular embodiment. However, the portion of said means 13 which is within the core 3 may be situated away from the back sheet 2, for example at a location adjacent the upper surface 14 of said core 3.

The indicator means 13 in the embodiment illustrated comprises a detector portion 4 and an indicator portion 6, joined by a coupling transport portion or connecting line 5. Surrounding the detector portion 4 is a gate means 20 in the form of a layer or coating of dissolvable material. As will be described below, such a gate means 20 is constituted by a terpolymer coating, but other dissolvable layers may be used in addition to a terpolymer.

The detector portion 4 and gate means 20 are thus positioned in a part of the absorbent core most likely to be affected by exudate, whereby the gate means prevents access of the exudate to the detector until the gate means has changed state (in this example by the gate means dissolving sufficiently for the exudate to contact the detector portion 4). A suitable location for the detector portion 4 and gate means 20 is thus in the crotch portion of the absorbent product, close to the middle portion thereof (as shown). In this way, urine will have a high probability of reaching the gate means 20 and also later the detector portion 4 once the gate means has changed to its second state.

As a general rule, the detector portion will be located inside the region of the absorbent core, even if not always at its centre, whereby the detector will be able to be proximate to exudate. Differences of location may occur between male and female absorbent products. The "region of the absorbent core" should be understood as being the general vicinity of the absorbent core. Thus, a detector portion 4 and gate means 20 located totally inside the absorbent core will clearly be in this "region", but also placement of the detector on one of the surfaces of the core or on a top or back sheet, and thus immediately adjacent to the core and within the plan view outline of the core, is intended to be encompassed by the expression "region of the absorbent core".

The detector portion and its associated gate means 20 could of course be given other shapes and forms and be located at another part of the core or even at several different parts of the absorbent core, to provide more probability of urine reaching the detector portion 4. In a preferred embodiment, the detector portion 4 is positioned between the top sheet and the core, i.e. on top of the core on the side close to the wearer, where urine and faeces are mixed together.

The detector portion 4 is shown as being something different to the indicator portion 6 and spaced therefrom, although it should be understood that the detector portion may itself function as the indicator portion in certain embodiments. In this way no separate indicator portion is required. The indicator portion and detector portion can even be the same thing.

The gate means 20 is shown as covering the detector portion 4, although it will be evident that for the gate means to operate, it must merely be able to prevent activation of the indication means 13 until required. Thus, the gate means could equally be placed anywhere in the path starting with the detector portion 4 right up to the indicator portion 6, or indeed over at least part of the indicator portion 6 itself, as long as it would be in a position where it would be able to come into contact with exudate or a product of same.

The indicator portion 6 is intended to comprise any type of means that can provide a detectable indication, such as a visual indication, an audible indication, an indication by means of a specific fragrance, or even an indication by means of change of shape (e.g. for detection of a change of shape by blind persons), or even a combination of these indications.

In a preferred embodiment, in view of providing an indicator means which is cheap and can thus be used for mass production in disposable absorbent products, a visual indication is deemed most preferable. Thus in the embodiment of Fig. 1 the indicator portion 6 has been shown positioned in a relatively easily visible position on the diaper, approximately midway between the strips 7 and 8, where it is unlikely to be obscured by the fastening of strips 9 and 10 thereto. However, the indicator portion may be positioned at any suitable location, such as close to the crotch portion of the article, although it will be appreciated that such a location may make inspection of same somewhat more burdensome, but at the same time it would have the advantage of being cheaper and smaller overall than the indicator means shown in Fig. 1. Similarly the indicator means could have at least a part thereof positioned outside the wearer's normal clothing, in which case it would typically be attached by a physical communicating means to the detector portion and gate means inside the absorbent product, although it would be possible to construct the detector portion to include a radio transmitter and the indicator portion to include a receiver, thereby allowing the radio receiver portion to be a re-usable item. The gate means in such a case could for example be an electronic component of some type within the diaper which provided a threshold signal level required for the detector to be able to transmit.

When the indicator portion 6 and the detector portion 4 are spaced apart, a connection must be established therebetween. In the embodiment shown in Fig. 1 this is provided by a connection or transport means 5. The transport means 5 serves to activate the indicator portion 6 upon the detector portion 4 detecting a required parameter, which activation is enabled when the gate means 20 changes state. In the present invention, one parameter which is seen as being an important one to be detected is the pH value present in the fluid, normally urine, in an absorbent product.

Since pH level is indeed used as the threshold, the indicator means and the gate means should provide a gate system able to detect, selectively, pH of specific threshold values. For example, a selective pH of substantially 6.5 and above in the exudate would not be susceptible to values below about 6.5. Where the detection of urine and faeces together is desired, pH values of about 7.0 and above might also be used as the detection threshold, since this is even more easily distinguishable from some people's urine pH value which can be present dependent on the condition of the wearer.

The gate means for use with pH threshold gate systems of the invention is a terpolymer. Terpolymers are used traditionally to provide enteric coatings for pharmaceutical compositions which can be broken down by intestinal fluids (see e.g. US-A-4 704 295). Terpolymers are also used in the field of detergent compositions for cleaning (see e.g. US-A-5 000 869). However it has now been appreciated by the inventors that terpolymers can be usefully employed in the present invention as a gate means which can change state so as to provide a threshold of pH and thereby also an accurate indication of pH.

Terpolymers basically comprise three components, a temperature sensitive component, a polymer backbone (i.e. filler) and a pH sensitive component. In particular, terpolymers are formed by acid-ionisation such that they remain insoluble at lower pH values, depending on the ionisation level. This feature is useable in the present invention, since the lowest pH at which the terpolymer dissolves can be arranged within fine limits to a specific pH value. The techniques for fixing this pH value or set of pH values are readily known in the art of terpolymer construction and thus require no detailed description here.

In the embodiment of Figs. 1 and 2, the indicator portion 6 of the indicator means 13 may be constituted by a colour changing strip which is visible through the back sheet 2. As a further alternative, a highly transparent and fluid impervious window could be arranged in the back sheet.

Although the embodiment of Fig.1 shows separate detector and indicator portions 4 and 6, the whole indicator means itself may be formed by a pH indicating strip, whereby the gate means 20 would then have to cover the whole indicator means so as to prevent any part changing colour until the gate threshold was reached.

A paper-based strip could be used to advantage since the capillary pressure in the paper will help to draw fluid from the core along the paper into the outerlying portions of same, such as by drawing fluid from the core centre and up to the waistband. Such a strip could also be covered by an impermeable coating (not shown) along the majority of its length and thus have a gate means only over one end thereof. In this way, fluid entry into the paper-based strip would only be allowed to occur at one location and only when the gate means has changed state.

As a further alternative, the detector portion 4 can be arranged to cause activation of the indicator portion 6, by sending a signal of some type (e.g. a colour signal, electrical signal, fluid signal etc.) by means of a connection portion or line 5, whereby the gate means 20 would have to be placed in a suitable position to block said signal.

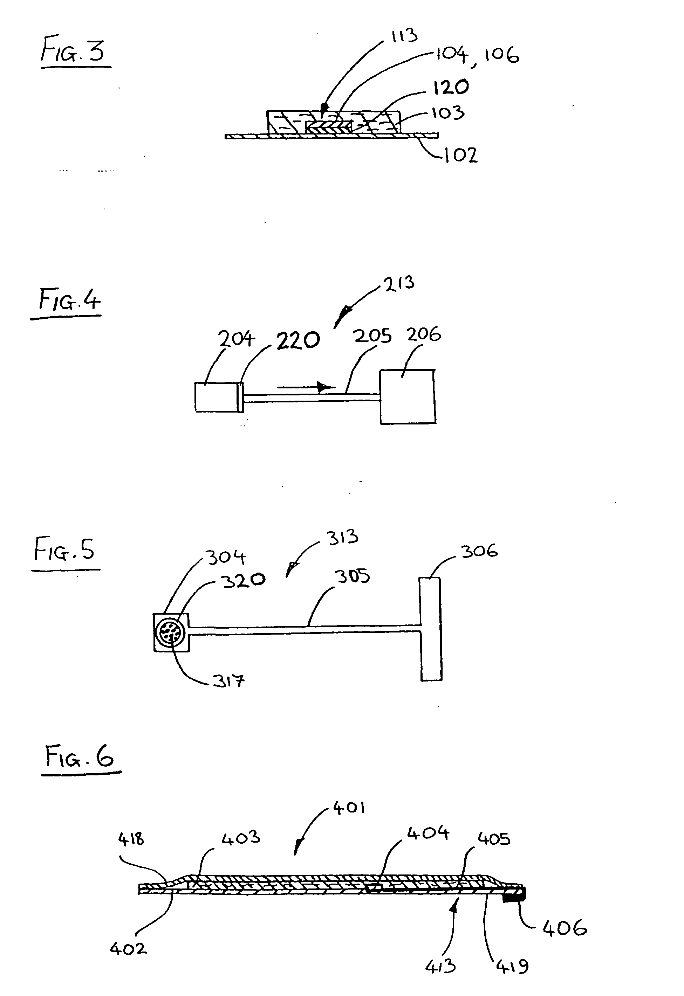

Fig. 3 shows a further embodiment of the indicator means 13, similar to the sectional view shown in Fig. 2, whereby like features have like reference numerals raised by 100. In Fig. 3, the indicator portion 106 (and commensurate detector portion 104) is separated from the back sheet by an intermediary covering layer. In this case the covering layer constitutes a gate means 120 which is dissolvable at the required pH threshold or pH and time threshold.

A terpolymer arranged to dissolve at pH of e.g. about 6.5 and above, could be used as a suitable gate means 120. When the gate means 120 is itself the pH-sensitive means, the indicator portion 106 may then be an "inert" indicator, such as a strip of a permanent single bright fluorescent yellow colour, whereby when the gate means 120 formed by the covering layer is dissolved or broken away, the fluorescent colour becomes clearly visible through the back sheet 102, but does not itself undergo any change.

The gate means may also be in the form of a substance which is opaque until a certain threshold is reached and then changes state to be transparent, thereby providing visual access to a visible indicator underneath the gate means for example.

Additionally to the gate means 120 changing state to allow the underlying indicator to become visible, the indicator means 13 could itself also be colour changeable at or above the required pH, and protected entirely by the gate means in the form of a covering layer (e.g. the aforementioned terpolymer). In this way, the gate means 120 could provide a first barrier to the indicator portion 106 being visible and, when the gate changes state (i.e. the terpolymer dissolves) as a result of its contact with exudate, the colour changeable indicator could be allowed to come into contact with the exudate substance having a pH of e.g. 6.5 and above and thus change colour. This could be used to provide a dual-level indicator system, whereby the covering layer gate means (terpolymer) breaks down and dissolves at e.g. pH 6.5 to reveal the underlying indicator portion 106 in one colour, whereas when the pH rises to 7.0 or a higher value, the indicator can assume a further colour which is also visible. In this way, the wearer or attendant can be warned at a first pH level (caused by the terpolymer dissolving for example) that harmful conditions exist and then at a second level (caused by the indicator portion 6 colour change) that the pH has risen to a new higher level (e.g. 7.0 or 8.0) such that rapid degradation of the skin is likely to occur unless the absorbent product is changed soon.

Although in Fig. 3, the gate means 120 (terpolymer) is shown as being a coating on only one side of the indicator means 13 (i.e. elements 104 and 106 in this embodiment), the indicator means 13 (or any specific portion(s) thereof) may be entirely enclosed in the gate means 120 material on its entire surface. Similarly, the gate means 120 is shown as being coextensive with the combined indicator and detector portions 106 and 104, but could of course be wider and longer than same so as to prevent the side portions of same being seen through the back sheet until the gate means has changed state.

The thickness of the gate means 120 depends on the predetermined time threshold which is desired before visual indication should take place after the lowest selected pH level threshold has been reached. This may be used advantageously so as to avoid warning the wearer or attendant of a urine pH value which is possibly high and undesirable, but which at the same time is so small that it is entirely absorbed in the lower part of the core 3 (i.e. part closest to the back sheet 2 during use) and thus of relatively insignificant danger for the wearer's skin. A thicker layer will evidently take more time to dissolve than a thinner layer. Thus, where the time threshold is wished to be very low (e.g. a few seconds), or substantially zero, only a very thin coating of terpolymer is necessary.

Fig. 4 illustrates a sketch of a further possibility whereby the gate means 220 (formed by the terpolymer) prevents any signal produced by the detector 204 from reaching the connector or the signal transport means 205, to thereby inhibit the indicator portion 206 from being activated to produce an indication. For example, detector 204 may provide a source of coloured dye that is to be transported to indicator portion 206 by means of a connection line 205 (e.g. a thin capillary filled with water), whereby the gate 220 first needs to be changed to its second state to allow movement of the dye down the line 205. This change to the second state can be as a result of exceeding a specific pH threshold or a pH and contact time threshold for example. The terpolymer gate 220 could also be applied as a coating over the detector portion 204.

Instead of a source of coloured dye, detector 204 may instead contain a first active substance or composition which, when released, can react with a second active substance or composition in the presence of liquid in the line 205 to produce a colour change visible in for example line 205 and/or indicator portion 206. Since there are many possibilities of chemicals which can produce such a colour change which are known to the skilled person, further discussion of same here is unnecessary.

Similarly, line 205 could be an electrical conductor transferring a current generated by detector portion 204 as a result of pH level being reached, whereby gate means 220 is in the form of a switch. Such a switch could prevent current flow below a predetermined threshold, but when opened (e.g. by removal thereof) allows a current flow to activate indicator portion 206 which can give an audible and/or visual indication. Detector portion 204 and gate means 220 can of course be an integral unit or in fact the same thing. Fig. 4 is thus merely illustrative of the principle of operation and is not intended to dictate the exact positional interrelationship of the various elements, but merely indicates a possible position of the gate means 220 with respect to the detector and indicator.

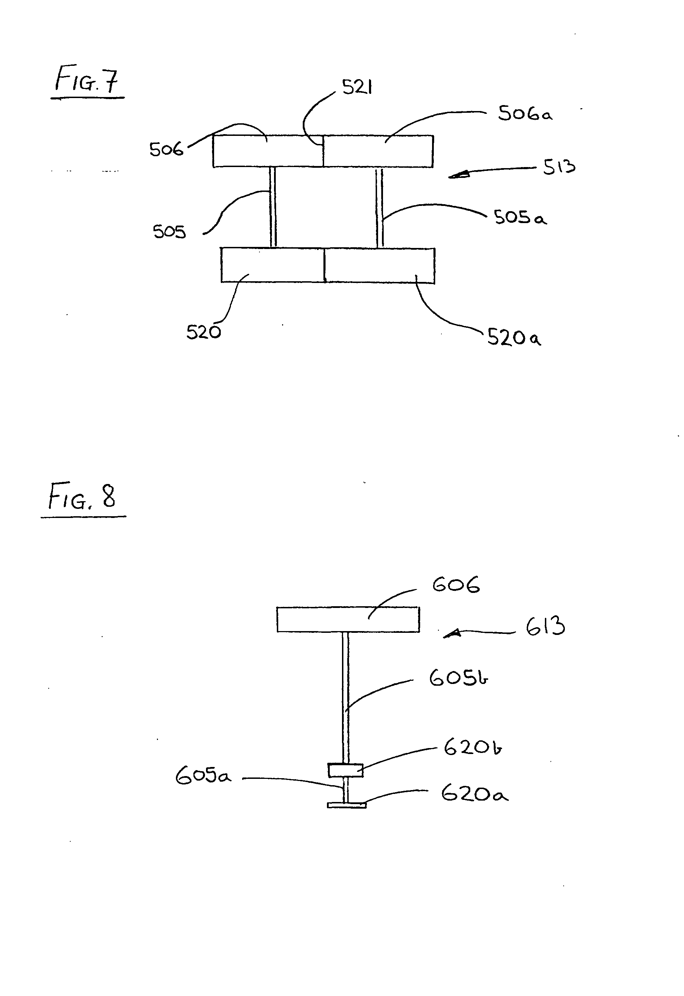

Fig. 5 shows an embodiment similar to Fig. 4, whereby a coloured dye 317 is stored in an encapsulation of a pH sensitive blocker material constituting a gate means 320 as part of detector portion 304. Upon breakdown of the blocker material of the gate means 320 (i.e. the terpolymer), the dye 317 is allowed to travel along path 305 to thereby be displayed by the indicator portion 306. The detector portion 304 and the connector line 305 can thus be made of an opaque material, whilst leaving the indicator portion 306 transparent. Alternatively, the entire indicator means 313 could have a single base material which is stainable all over when the dye 317 is released.

As an alternative (not shown), the dye 317 might be replaced by, or supplemented with, a foam substance which when activated due to the gate means changing to its second state, causes foam to be formed at a fast rate and thereby fill an expandable cavity in indicator means 306 (or in portion 304 if portions 304 and 306 are combined at the same location). In this way, a change of shape can constitute the indication, whereby the indicator means 306 would be placed in a location where the change of shape could be easily felt by the user. This may be suitable for users who are colour-blind or have defective sight. For example, the indicator means may provide a smooth surface having weakened expandable portions in the normal state, whereby filling the indicator means 306 with foam or gas or the like (as a result of pH above 6.5 for example) would expand said weakened portions and thus change the texture of the smooth surface so as to be recognisably different (visually and/or by touch) to the surface in its original state.

Although a terpolymer has been proposed in a particular embodiment, other possibilities exist which may be used for other gates. A further possibility is for example a pH sensitive hydrogel. Carboxylated hydrogels are particularly suitable since they are non-toxic, cheap and present a sharp pH shift, thus allowing high accuracy pH to be used as the threshold in the present invention. pH-sensitive hydrogels change state by swelling in such a way that they become permeable to both small and large molecules. The critical value of pH at which a hydrogel will swell is dependent on the pKa value of the acid moieties of the hydrogel. In this way, the skilled person can provide a pH threshold value adjusted to swell at the required pH.

Thus, taking the embodiment of Fig. 4 for example, where, in addition to the terpolymer gate of the invention, a gate 220 described above can be formed by a hydrogel which initially is plugged by large molecules which are freed, at the required pH, due to the swelling of the hydrogel. The freeing of the large molecules opens one or more channels through which the contents of part 204 comes into contact with the contents of part 205. This can likewise be applied to the embodiment of Fig. 5 by construction of the gate 320 as a hydrogel carrying large molecules which upon swelling are freed to open channels allowing dye 317 (or other component) to pass through the gate 320.

A further alternative for a gate in addition to the terpolymer gate of the invention is to use any one of many known mildly acidic salts which will saturate at low pH levels and dissolve freely in alkali conditions. Depending on the pH dissolution threshold level required, it is merely a matter of selecting a salt e.g. from the Merck Index listing of strong acids and weak bases (e.g. calcium sulphate salt or the like). This selection is not described in further detail here since the selection of specific salts is not a limitation of the present invention. It may also be mentioned that there are also any number of low-molecular weight organic acid salts which are readily available having a wide range of dissolution points.

A still further possibility in addition to the terpolymer gate of the invention is the use of chitosan as the blocking material or layer. Chitosan is namely soluble in dilute aqueous organic acids (thus including values for example pH values between 6.5 to 7.0) and thus applicable for use as a gate in the invention. Chitosan is namely not soluble in many other solutions.

Although dissolution of the gate of blocking material upon the required pH threshold value being reached has been described in some of the above embodiments, further possibilities include constructing other gates from a compound which exhibits a marked change in viscosity at a particular threshold of pH. For example, proteins have no significant shift in solubility as pH varies since they are highly amphoteric. However, proteins do show a shift in viscosity value or gel point with pH which can be sufficient for use as an additional pH gate to the terpolymer gate of this invention. For example, taking the embodiment of Fig. 4, the line 205 may be constructed as a capillary filled with fluid, which capillary can be plugged with a highly viscous polymeric gel which becomes less viscous at the required pH (e.g. at pH 6.5 and above, 6.7 and above, etc.). This then allows the plug to be freed at the required pH (i.e. the gate is opened). The selection of the requisite polymeric gel is not described in further detail here, since the specific polymer can simply be selected according to the pH value required.

Fig. 6 shows an arrangement of an indicator means 413 having a detector portion 404, connection line 405 and indicator portion 406, whereby the indicator portion 406 lies on or adjacent to the external surface 418 of the back sheet 402, whilst the detector portion 404 and part of line 405 may be arranged between the top sheet 418 and back sheet 402, the detector portion being in or adjacent to the absorbent core 403. In this way, perception of the indicator portion is facilitated, be it audio, visual or a combination of any number of perceivable indications. The gate means (not shown in this embodiment) might be suitably positioned around the detector portion 404, such that the gate means (i.e. the terpolymer part which needs to contact the exudate) is entirely inside the absorbent core 403.

Fig. 7 illustrates an embodiment of parallel gate means 520, 520a connected by means of connection lines 505, 505a to indicators 506, 506a respectively. The arrangement depicted may be for example with the gates means 520, 520a in an absorbent core (not shown) and the indicator means 506, 506a being in a visible position on the absorbent product (e.g. at the waist band of a diaper). Indicator 506 may for example be red at pH values below about 5.0 and change to a blue colour at pH values of between about 5.0 and 8.0. Such an indicator could be a 2.5-dinitrophenol indicator. Similarly indicator 506a might be an indicator which changes colour from yellow to purple above about pH 4.8 and up to 6.4 (such as a cholorophenol red indicator). Other ranges may be chosen depending on what indication is required. Connection lines 505, 505a may be constituted by narrow capillaries in which urine will flow quickly to activate the indicators 506, 506a respectively when gate means 520, 520a have changed state. The gate means 520, 520a may each be constituted by a terpolymer and capable of dissolving at pH values of 5.5 and above and 6.5 and above respectively. Thus when gate 520 changes state by dissolving above the required pH threshold, it allows activation by urine of the first indicator 506 so that this will change colour to blue. If the pH is below 6.5, it will however not dissolve gate 520a and thus no colour change will occur at indicator 506a. In this way, an indication of wetness due to urine is made, but the pH level does not imply that an urgent change of absorbent product is needed. If however, the purple colour appears on indicator portion 506a, the attendant is warned that skin damage may occur if a change does not occur soon.

From the above it is clear that although the pH ranges set by the individual colour indicators themselves are relatively broad, the gate means 520, 520a is capable of providing a much greater level of accuracy in pH evaluation and thus better information to the attendant/wearer.

While the above embodiment is shown with only two indicators in parallel, it should be understood that more indicators can also be used in parallel and/or in series. Thus, a whole range of gates and indicators could for example be provided at differing pH values, such as pH 6.5, 6.7, 6.9, 7.1, 7.3, 7.5 etc.

Similarly, the elements 506 and 506a are shown as joined in the form of a strip. The indicators do not however need to be physically interconnected but could be separate. Preferably the indication area would be kept to a minimum and thus a small strip of indicator segments (i.e. similar to indicator portions 506, 506a) would provide a suitable means of display.

Furthermore whilst the indicators in the above embodiment have been described as being constituted by different colour changes, the same colour changes can be used (e.g. red to blue litmus indicator), whereby when the first gate means 520 changes state (e.g. at urine of about pH 5.8 for example) the portion 506 will turn blue, but the second indicator 506a will not change to blue until the higher specific pH threshold is reached. In this way, an attendant can also be informed of the urgency of an absorbent product change by seeing how much of the combined strip 506, 506a has changed colour. If the strip 506, 506a would be a paper strip, a separation means 521 of some type (e.g. an adhesive line within the strip) would be useful to stop one part of the strip affecting the other and thus effectively circumventing the gate means.

Figure 8 depicts an embodiment of a gate system with two gate means 620a, 620b in series, which gate means are joined by a connection line 605a. Similarly, connection line 605b joins gate means 620b to indicator portion 606. Gates 620a, 620b may be positioned appropriately in, or in the vicinity of, an absorbent core in a manner similar to that shown in Fig. 1 for example. Taking an exemplary embodiment, a gate means 620a is provided which may be in the form of a very thin terpolymer layer dissolvable within a few seconds of contact with a solution of a selected lower pH threshold (e.g. pH 6.5) to thereby open connection line 605a to allow passage of liquid (e.g. urine) to gate 620b. Likewise, second gate means 620b may be a substance (e.g. a further terpolymer) dissolvable after a specific pH level is exceeded, for example at a pH above 4.5 and preferably at a level lower than said first pH level. The thickness of the second gate means however can be chosen such that a selected time period (e.g. a number of minutes) is required before the gate means changes to its second state.

As can be understood, the choice of gate means can be chosen to suit the particular parameter evaluation that is required for activation of the indicator means.

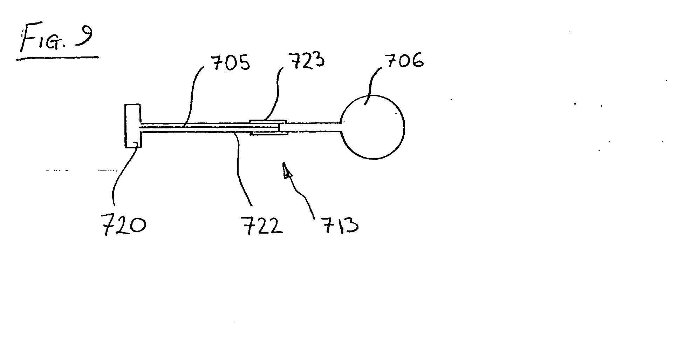

A further embodiment for use together with a terpolymer gate of the invention is illustrated schematically in Fig. 9 and concerns the use of a gas threshold gate means 720 connected to an indicator such as an audible indicator 706 like a buzzer alarm. Both alarm and gate means 720 can be positioned easily within the absorbent product and may be connected by a conductor 705 within a covering of plastic 722 for example. Preferably however, the gate means and indicator means could be formed as a single combined unit and be located entirely in the diaper. The indicator may however be a reusable unit connected by simple sleeve 723 to the connection line 705.

The gate means 720 in such a case could be a cumulative gas detector which remains in a passive state until a particular gas concentration is reached in its location, whereupon the gate can alter state such that a signal is sent to indicator 706 to activate same. A high gas concentration will result in a faster change of the gas detector, since a gas quantity threshold is reached faster. By including such a gas concentration gate means within the absorbent product (such as at a position above the location where the anus would be located), mercaptan or amine gases produced as a result of the presence of faeces can be detected.

Other gases can also be detected, for example by providing a substance in the absorbent product which will release a specific gas upon contact with urine and/or faeces for example. In this way, the gas detector can be arranged not only to detect mercaptans or amines but any other suitable gas.

Further embodiments will be readily understood by the skilled person upon reading the aforegoing and are intended to be encompassed within the scope of the invention as defined by the appended claims.

## Claims

1. A gate system for an absorbent product (1) for absorbing bodily exudate, comprising a gate means (20; 120; 220; 320; 520, 620a, 620b; 720) coupled to at least a part of an indicator means (13; 113; 213; 313; 413; 513; 613; 713), wherein said gate means can change from a first state preventing activation of said indicator means (13; 113; 213; 313; 413; 513; 613; 713) to a second state allowing activation of said indicator means, wherein said gate means (20; 120; 220; 320; 520, 620a, 620b; 720) is actuated by contact between itself and body exudate or between itself and a product of body exudate, and in that said gate means changes to said second state after a predetermined threshold resulting from said contact is exceeded, and wherein said gate means is changeable into said second state by being dissolvable as a result of contact with an exudate or product thereof, to thereby allow activation of said indicator portion (6; 106; 206; 306; 406; 506, 506a; 606), **characterized in that** said gate means (20; 120; 220; 320; 520, 520a; 620a, 620b) comprises a pH-sensitive terpolymer, said terpolymer including a temperature sensitive component, a polymer backbone and a pH sensitive component, said terpolymer remaining in its first state when said pH level is below a predetermined pH threshold, and being changeable into said second state by being dissolvable above said pH threshold.

2. A gate system according to claim 1, **characterized in that** the predetermined pH threshold of the gate means (20; 120; 220; 320; 520, 620a, 620b) is a selected pH threshold and is combined with a time threshold.

3. A gate system according to claim 1 or claim 2, **characterized in that** said indicator means (13; 113; 213; 313; 413; 513; 613; 713) comprises a detector portion (104; 204; 304; 404; 504) and an indicator portion (6; 106; 206; 306; 406; 506, 506a; 606; 706), and **in that** said gate means (20; 120; 220; 320; 520, 620a, 620b; 720) prevents activation of said indicator portion by preventing contact of said detector portion and/or said indicator portion with body exudate or a product thereof.

4. A gate system according to claim 3, **characterized in that** said detector portion (204; 304; 404) is spaced from said indicator portion (206; 306; 406) to allow detection at a different location to indication.

5. A gate system according to any one of the preceding claims, **characterized in that** said terpolymer is applied as a coating over said indicator portion (106) whereby said terpolymer obscures said indicator portion (106) from view until dissolved.

6. A gate system according to claim 5, **characterized in that** said terpolymer (220) is applied as a coating over said detector portion (204).

7. A gate system according to any one of the preceding claims, **characterized in that** said gate means (20; 120; 220; 320; 520, 620a, 620b; 720) is attached inside an absorbent product (1), said absorbent product having an absorbent core (3; 103; 403).

8. A gate system according to claim 7, **characterized in that** said gate means is attached inside the region of said absorbent core (3; 103; 403).

9. A gate system according to any one of the preceding claims, **characterized in that** said absorbent product (1) is a diaper or a pair of absorbent pants.

10. A gate system according to any one of the preceding claims, **characterized in that** said system includes a plurality of gate means (520, 520a), whereby each of said gate means has its own predetermined threshold level required for changing to said second state, and **in that** said gate means are arranged in parallel to thereby allow individual activation of a separate indicator means (506, 506a) as the predetermined threshold of a respective gate means (520, 520a) is exceeded.

11. A gate system according to any one of claims 1 to 9, **characterized in that** a plurality of gate means (620a, 620b) is included, and **in that** said gate means are arranged in series such that a first one of said gate means (620a) needs to change to its second state before a further gate means (620b) is exposed.

12. A gate system according to claim 10 or 11, **characterized in that** each of said gate means (520, 520a; 620a, 620b) has a different predetermined pH threshold.

13. A gate system according to claim 10 or 11, **characterized in that** each of said gate means (520, 520a; 620a, 620b) has a different predetermined time threshold.

14. A gate system according to claim 12 or 13, **characterized in that** each of said gate means, in its second state, allows activation of a visible colour indicator portion (506, 506a) of different colour.

## Patentansprüche

1. Schrankensystem für ein absorbierendes Produkt (1) zum Absorbieren von Körperexsudat, umfassend eine Schrankeneinrichtung (20; 120; 220; 320; 520; 620a, 620b; 720), die mit wenigstens einem Teil einer Indikatoreinrichtung (13; 113; 213; 313; 413; 513; 613; 713) gekoppelt ist, wobei die Schrankeneinrichtung von einem ersten Zustand, der die Aktivierung der Indikatoreinrichtung (13; 113; 213; 313; 413; 513; 613; 713) verhindert in einen zweiten Zustand, der die Aktivierung der Indikatoreinrichtung zulässt, wechseln kann, wobei die Schrankeneinrichtung (20; 120; 220; 320; 520; 620a, 620b; 720) durch Kontakt zwischen ihr und einem Körperexsudat oder zwischen ihr und einem Produkt des Körperexsudats betätigt wird und wobei die Schrankeneinrichtung in den besagten zweiten Zustand wechselt, nachdem ein vorbestimmter Grenzwert, der auf den Kontakt folgt, überschritten wird, und wobei die Schrankeneinrichtung derart in den zweiten Zustand wechseln kann, dass sie als eine Folge des Kontakts mit einem Exsudat oder einem Produkt davon löslich ist, um dadurch die Aktivierung des Indikatorabschnitts (6; 106; 206; 306; 406; 506; 506a, 606) zu gestatten, **dadurch gekennzeichnet, dass** die Schrankeneinrichtung (20; 120; 220; 320; 520; 520a, 620a; 620b) ein pH-sensitives Terpolymer umfasst, wobei das Terpolymer eine temperatursensitive Komponente, ein Polymerbasisnetz und eine pH-sensitive Komponente enthält, wobei das Terpolymer in seinem ersten Zustand bleibt, wenn der pH-Wert unter einen vorbestimmten pH-Grenzwert liegt und dadurch, dass er dadurch in den zweiten Zustand wechseln kann, dass er über dem pH-Grenzwert löslich ist.

2. Schrankensystem nach Anspruch 1, **dadurch gekennzeichnet, dass** der vorbestimmte pH-Grenzwert der Schrankeneinrichtung (20; 120; 220; 320; 520; 620a, 620b) ein gewählter pH-Grenzwert ist und mit einem Zeitgrenzwert kombiniert ist.

3. Schrankensystem nach Anspruch 1 oder 2, **dadurch gekennzeichnet, dass** die Indikatoreinrichtung (13; 113; 213; 313; 413; 513; 613; 713) einen Erfassungsabschnitt (104; 204; 304; 404; 504) und einen Indikatorabschnitt (6; 106; 206; 306; 406; 506; 506a; 606; 706) umfasst und dadurch, dass die Schrankeneinrichtung (20; 120; 220; 320; 520; 620a, 620b; 720) die Aktivierung des Indikatorabschnitts dadurch verhindert, dass ein Kontakt des Erfassungsabschnitts und/oder des Indikatorabschnitts mit einem Körperexsudat oder einem Produkt davon verhindert wird.

4. Schrankensystem nach Anspruch 3, **dadurch gekennzeichnet, dass** der Erfassungsabschnitt (204; 304; 404) von dem Indikatorabschnitt (206; 306; 406) beabstandet ist, um zur Indikation eine Erfassung an unterschiedlichen Stellen zu gestatten.

5. Schrankensystem nach einem der vorstehenden Ansprüche, **dadurch gekennzeichnet, dass** das Terpolymer als eine Beschichtung über dem Indikatorabschnitt (106) aufgetragen ist, wobei das Terpolymer die Sicht auf den Indikatorabschnitt (106) verhindert, bis er aufgelöst ist.

6. Schrankensystem nach Anspruch 5, **dadurch gekennzeichnet, dass** das Terpolymer (220) als eine Beschichtung über den Erfassungsabschnitt (204) aufgetragen ist.

7. Schrankensystem nach einem der vorstehenden Ansprüche, **dadurch gekennzeichnet, dass** die Schrankeneinrichtung (20; 120; 220; 320; 520; 620a, 620b; 720) in einem absorbierenden Produkt (1) angebracht ist, wobei das absorbierende Produkt einen Absorptionskern (3; 103; 403) aufweist.

8. Schrankensystem nach Anspruch 7, **dadurch gekennzeichnet, dass** die Schrankeneinrichtung innerhalb des Bereichs des Absorptionskerns (3; 103; 403) angebracht ist.

9. Schrankensystem nach einem der vorstehenden Ansprüche, **dadurch gekennzeichnet, dass** das absorbierende Produkt (1) eine Windel oder ein absorbierendes Höschen ist.

10. Schrankensystem nach einem der vorstehenden Ansprüche, **dadurch gekennzeichnet, dass** das System mehrere Schrankeneinrichtungen (520, 520a) umfasst, wobei jede der Schrankeneinrichtungen ihren eigenen vorbestimmten Grenzwert aufweist, der erforderlich ist, um in den zweiten Zustand zu wechseln und dadurch, dass die Schrankeneinrichtungen parallel angeordnet sind, um dabei eine individuelle Aktivierung einer separaten Indikatoreinrichtung (506, 506a) zu gestatten, wenn der vorbestimmte Grenzwert einer entsprechenden Schrankeneinrichtung (520, 520a) überschritten ist.

11. Schrankensystem nach einem der Ansprüche 1 bis 9, **dadurch gekennzeichnet, dass** mehrere Schrankeneinrichtungen (620a, 620b) enthalten sind und dadurch, dass die Schrankeneinrichtungen in Reihe angeordnet sind, so dass eine erste der Schrankeneinrichtungen (620a) in den zweiten Zustand wechseln muss, bevor eine weitere Schrankeneinrichtung (620b) freigelegt wird.

12. Schrankensystem nach Anspruch 10 oder 11, **dadurch gekennzeichnet, dass** jede Schrankeneinrichtung (520, 520a, 620a, 620b) einen anderen vorbestimmten pH-Grenzwert aufweist.

13. Schrankensystem nach Anspruch 10 oder 11, **dadurch gekennzeichnet, dass** jede Schrankeneinrichtung (520, 520a; 620a, 620b) einen anderen vorbestimmten Zeitgrenzwert aufweist.

14. Schrankensystem nach Anspruch 12 oder 13, **dadurch gekennzeichnet, dass** jede Schrankeneinrichtung in ihrem zweiten Zustand die Aktivierung eines sichtbaren Farbindikatorabschnitts (506, 506a) mit unterschiedlicher Farbe gestattet.

## Revendications

1. Système de barrière pour un produit absorbant (1) pour absorber des exsudats corporels, comprenant un moyen formant barrière (20 ; 120 ; 220 ; 320 ; 520, 620a, 620b ; 720) couplé à au moins une partie d'un moyen indicateur (13 ; 113 ; 213 ; 313 ; 413 ; 513 ; 613 ; 713), dans lequel ledit moyen formant barrière peut passer d'un premier état où l'activation dudit moyen indicateur (13 ; 113 ; 213 ; 313 ; 413 ; 513 ; 613 ; 713) est empêchée à un deuxième état où l'activation dudit moyen indicateur est autorisée, dans lequel ledit moyen formant barrière (20 ; 120 ; 220 ; 320 ; 520, 620a, 620b ; 720) est actionné par contact entre lui-même et un exsudat corporel ou entre lui-même et un produit d'exsudat corporel, et où ledit moyen formant barrière passe audit deuxième état après qu'un seuil prédéterminé a été dépassé suite audit contact, et dans lequel ledit moyen formant barrière peut passer dans ledit deuxième état en devenant soluble à la suite du contact avec un exsudat ou avec un produit de celui-ci, pour permettre ainsi l'activation de ladite partie indicateur (6 ; 106 ; 206 ; 306 ; 406 ; 506, 506a ; 606), **caractérisé en ce que** ledit moyen formant barrière (20 ; 120 ; 220 ; 320 ; 520, 520a ; 620a, 620b) comprend un terpolymère sensible au pH, ledit terpolymère incluant un composant sensible à la température, un squelette polymère et un composant sensible au pH, lequel terpolymère reste dans son premier état lorsque ledit niveau de pH se trouve sous un seuil de pH prédéterminé, et peut passer audit deuxième état en devenant soluble au-dessus dudit seuil de pH.

2. Système de barrière selon la revendication 1, **caractérisé en ce que** le seuil de pH prédéterminé du moyen formant barrière (20 ; 120 ; 220 ; 320 ; 520, 620a, 620b) est un seuil de pH sélectionné et est associé à un seuil de temps.

3. Système de barrière selon la revendication 1 ou 2, **caractérisé en ce que** ledit moyen indicateur (13 ; 113 ; 213 ; 313 ; 413 ; 513 ; 613 ; 713) comprend une partie détecteur (104 ; 204 ; 304 ; 404 ; 504) et une partie indicateur (6 ; 106 ; 206 ; 306 ; 406 ; 506, 506a ; 606 ; 706), et **en ce que** ledit moyen formant barrière (20 ; 120 ; 220 ; 320 ; 520, 620a, 620b ; 720) empêche l'activation de ladite partie indicateur en empêchant le contact de ladite partie détecteur et/ou ladite partie indicateur avec un exsudat ou un produit de celui-ci.

4. Système de barrière selon la revendication 3, **caractérisé en ce que** ladite partie détecteur (204 ; 304 ; 404) est espacée par rapport à ladite partie indicateur (206 ; 306 ; 406) pour permettre la détection en un endroit différent de celui de l'indication.

5. Système de barrière selon l'une quelconque des revendications précédentes, **caractérisé en ce que** ledit terpolymère est appliqué sous forme de revêtement sur ladite partie indicateur (106), grâce à quoi ledit terpolymère cache ladite partie indicateur (106) à la vue jusqu'à ce qu'il soit dissous.

6. Système de barrière selon la revendication 5, **caractérisé en ce que** ledit terpolymère (220) est appliqué sous forme de revêtement sur ladite partie détecteur (204).

7. Système de barrière selon l'une quelconque des revendications précédentes, **caractérisé en ce que** ledit moyen formant barrière (20 ; 120 ; 220 ; 320 ; 520, 620a, 620b ; 720) est attaché à l'intérieur d'un produit absorbant (1), ledit produit absorbant comportant un coeur absorbant (3 ; 103 ; 403).

8. Système de barrière selon la revendication 7, **caractérisé en ce que** ledit moyen formant barrière est attaché à l'intérieur de la région dudit coeur absorbant (3 ; 103 ; 403).

9. Système de barrière selon l'une quelconque des revendications précédentes, **caractérisé en ce que** ledit produit absorbant (1) est une couche-culotte ou une culotte absorbante.

10. Système de barrière selon l'une quelconque des revendications précédentes, **caractérisé en ce que** ledit système comprend plusieurs moyens formant barrières (520, 520a), chacun desdits moyens formant barrières ayant son propre niveau de seuil prédéterminé requis pour passer audit deuxième état, et **en ce que** lesdits moyens formant barrières sont placés en parallèle pour permettre ainsi l'activation individuelle d'un moyen indicateur distinct (506, 506a) lorsque le seuil prédéterminé d'un moyen formant barrière respectif (520, 520a) est dépassé.

11. Système de barrière selon l'une quelconque des revendications 1 à 9, **caractérisé en ce qu'**une pluralité de moyens formant barrières (620a, 620b) est incluse, et **en ce que** lesdits moyens formant barrières sont disposés en série, de sorte qu'un premier desdits moyens formant barrières (620a) doit passer dans son deuxième état pour qu'un moyen formant barrière suivant (620b) soit exposé.

12. Système de barrière selon la revendication 10 ou 11, **caractérisé en ce que** chacun desdits moyens formant barrières (520, 520a ; 620a, 620b) a un seuil de pH prédéterminé différent.

13. Système de barrière selon la revendication 10 ou 11, **caractérisé en ce que** chacun desdits moyens formant barrières (520, 520a ; 620a, 620b) a un seuil de temps prédéterminé différent.

14. Système de barrière selon la revendication 12 ou 13, **caractérisé en ce que** chacun desdits moyens formant barrières, dans son deuxième état, permet l'activation d'une partie indicateur en couleur visible (506, 506a) de couleur différente.
